(19)

Europäisches
Patentamt
European
Patent Office
Office européen
des brevets

(11) **EP 4 546 361 A1**

(12) **EUROPEAN PATENT APPLICATION**
published in accordance with Art. 153(4) EPC

(43) Date of publication:
**30.04.2025 Bulletin 2025/18**

(21) Application number: **23846966.2**

(22) Date of filing: **25.07.2023**

(51) International Patent Classification (IPC):
*G16H 20/30* (2018.01)   *G16H 50/50* (2018.01)
*G16H 50/20* (2018.01)   *G16H 50/70* (2018.01)
*A61B 5/349* (2021.01)   *A61B 5/024* (2006.01)
*A61B 5/22* (2006.01)   *G06N 3/045* (2023.01)
*G06N 3/08* (2023.01)

(52) Cooperative Patent Classification (CPC):
**A61B 5/024; A61B 5/22; A61B 5/349; G06N 3/045;
G06N 3/08; G16H 20/30; G16H 50/20; G16H 50/50;
G16H 50/70**

(86) International application number:
**PCT/KR2023/010755**

(87) International publication number:
**WO 2024/025308 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.07.2022 KR 20220094659**

(71) Applicant: **Medical AI Co., Ltd.**
**Seoul 06180 (KR)**

(72) Inventor: **KWON, Joonmyoung**
**Seoul 06180 (KR)**

(74) Representative: **Patentanwaltskanzlei
Matschnig & Forsthuber OG
Biberstraße 22
Postfach 36
1010 Wien (AT)**

(54) **METHOD FOR RECOMMENDING EXERCISE ON BASIS OF ELECTROCARDIOGRAM, AND COMPUTER PROGRAM RECORDED ON RECORDING MEDIUM IN ORDER TO EXECUTE SAME**

(57)   The present invention proposes a method of recommending exercise suitable for a user's real-time health condition based on an electrocardiogram. The method may include obtaining an electrocardiogram signal measured for a user, identifying the state of the cardiac pump of the user by analyzing the electrocardiogram signal using pre-trained artificial intelligence, and determining the type of exercise to be recommended to the user in accordance with the identified state of the cardiac pump. Through this, a user may receive a recommendation for exercise optimized for his or her current health condition simply by measuring his or her electrocardiogram.

FIG. 10

**Description**

**Technical Field**

The present invention relates to smart health care, and more particularly, to a method of recommending exercise suitable for a user's real-time health condition based on an electrocardiogram (ECG) and a computer program recorded on a storage medium in order to execute the method.

**Background Art**

An electrocardiogram is a graphical recording of visual changes in electrical activity generated by the heart muscles. Briefly, as for the conduction system of the heart, the sinoatrial node (the SA node) periodically generates an electrical signal to induce the contraction of the heart. When the electrical signal generated by the sinoatrial node (the SA node) is transmitted through the atrium, the atrioventricular node (the AV node) slightly delays the electrical signal and then transmits it. The electrical signal transmitted from the AV node spreads throughout the ventricle through the bundle of His and Purkinje fibers.

As for the waveforms of an electrocardiogram that can be identified according to the process of conduction of the heart, the P wave can be identified during the process in which as the atrium becomes polarized by the electrical signal generated by the sinoatrial node (the SA node), the heart muscles of the valve contract, and, as the atrium becomes depolarized again, the heart muscles of the valve relax again. The P-Q wave can be identified during the process in which the AV node delays the electrical signal so that the ventricle does not immediately respond when the atrium contracts. The QRS complex can be identified during the process in which the electrical signal delayed by the AV node is transmitted (Q), and thus, the ventricle becomes polarized (R) immediately and then depolarized (S) immediately. The S-T wave can be identified during the resting phase in which the electrical signal does not stimulate the heart so that the ventricle contracts and the blood in the ventricle moves to various parts of the body. Furthermore, the T wave can be identified during the process in which as the ventricle is weakly polarized and then depolarized again, the heart muscles of the ventricle and the valve relax simultaneously. Furthermore, in some cases, the U wave can be identified due to the repolarization of the intraventricular septum.

The order of appearance, shapes, sizes, and intervals of the waveforms (the P wave, the P-Q wave, the QRS wave, the S-T wave, the T wave, and the U wave) that can be identified through the electrocardiogram may change in various manners depending on the health condition. In other words, by analyzing the order of appearance, shapes, sizes, and intervals of the waveforms included in the electrocardiogram, a health condition can be inferred, and even the various causes that cause such a health condition can be inferred.

Meanwhile, an artificial neural network (ANN) is one of the detailed methodologies of machine learning, and refers to an algorithm that has a network form in which neurons, which are basic computational units corresponding to human nerve cells, are linked to one another. Representative artificial neural networks (ANNs) include a multilayer perceptron (MLP), a recurrent neural network (RNN), and a convolutional neural network (CNN).

More specifically, a multilayer perceptron (MLP) is an artificial neural network (ANN) that has a plurality of hidden layers between an input layer and an output layer. A recurrent neural network (RNN) is an artificial neural network (ANN) that has a recursive connection structure in which the output of neurons is input back to the input layer. Furthermore, a convolutional neural network (CNN) is an artificial neural network (ANN) that mimics the human optic nerve structure, and extracts a feature map from a plurality of convolutional layers, reduces the dimension of a matrix through subsampling, and extracts only important parts from the feature map.

On the other hand, the exercise refers to various physical activities performed for the purpose of maintaining or improving physical strength, performance, or health. Depending on the effect on the human body, exercise may be classified into aerobic exercise, anaerobic exercise, and flexibility exercise, and depending on the body part to be trained, exercise may be classified into muscle strengthening exercise, bone strengthening exercise, balance exercise, and multiple physical activities.

Exercise capacity refers to the degree of ability to exercise. In general, exercise capacity is evaluated based on a subject's age, gender, and presence or absence of a chronic disease. For this reason, conventional services that recommend or suggest exercise only recommend or suggest exercise in order from exercise having low impact on the user's body to exercise having high impact on the body, or simply recommend or suggest exercise that corresponds to age, gender, and a chronic disease.

However, the capacity of the heart, which is the factor that has the greatest influence on exercise capacity, may change from moment to moment depending on the previous action, metabolism, and stress. For example, in the case of a person who drank excessively the day before, exercise capacity may be significantly reduced due to decreased myocardial contractility and increased blood clots caused by dehydration. Furthermore, it is not practical for a non-medical person to perform an echocardiography test to check for exercise suitable for his or her health condition before starting exercise in his or her daily life.

Therefore, there is a need for a means that can recommend exercise suitable for a user's real-time health status without requiring the user to take one or more burdensome actions.

**Disclosure**

**Technical Problem**

One object of the present invention is to propose a method of recommending exercise suitable for a user's real-time health condition based on an electrocardiogram.

Another object of the present invention is to propose a computer program recorded on a storage medium in order to execute a method of recommending exercise suitable for a user's real-time health condition based on an electrocardiogram.

The objects of the present invention are not limited to the objects mentioned above, and other objects not mentioned will be clearly understood by those skilled in the art from the following description.

**Technical Solution**

In order to accomplish the above-described object, the present invention proposes a method of recommending exercise suitable for a user's real-time health condition based on an electrocardiogram. The method may include obtaining an electrocardiogram signal measured for a user, identifying the state of the cardiac pump of the user by analyzing the electrocardiogram signal using pre-trained artificial intelligence, and determining the type of exercise to be recommended to the user in accordance with the identified state of the cardiac pump.

The method may further include, after the determining the type of exercise, transmitting information, including the determined type of exercise, to user equipment (UE) set in advance in accordance with the user.

More specifically, the identifying may include: segmenting the electrocardiogram signal into a plurality of segmentation signals according to a time-series order; inputting the plurality of segmentation signals to the artificial intelligence, and obtaining a plurality of probability values from the artificial intelligence; and identifying the state of the cardiac pump of the user based on the plurality of obtained probability values.

In this case, the artificial intelligence may be configured to include a first artificial neural network (ANN) for preload corresponding to an end diastolic volume of a heart, a second ANN for afterload corresponding to resistance received by the heart while contracting, and a third ANN for myocardial contractility of the heart.

According to one embodiment, the first ANN may be trained based on displacements for the P wave, P-Q wave, and QRS wave included in each of the plurality of segmentation signals, the second ANN may be trained based on displacements for the QRS wave, S-T wave, and T wave included in each of the plurality of segmentation signals; and the third ANN may be trained based on displacements for all the waveforms included in each of the plurality of segmentation signals.

The identifying the state of the cardiac pump may include: identifying a heart rate based on the number of waveforms included in the electrocardiogram signal per unit time; determining the state value of the preload based on a plurality of probability values obtained from the first ANN; determining the state value of the afterload based on a plurality of probability values obtained from the second ANN; determining the state value of the myocardial contractility based on a plurality of probability values obtained from the third ANN; and identifying the state of the cardiac pump based on the heart rate, the state value of the preload, the state value of the afterload, and the state value of the myocardial contractility.

Meanwhile, the identifying the state of the cardiac pump may include identifying the user's maximum volume of oxygen uptake by analyzing the electrocardiogram signal using the artificial intelligence.

The determining the type of exercise may include determining the type of exercise matching the state of the cardiac pump of the user from a previously set exercise dictionary, and determining the intensity of the exercise matching the determined type of exercise and the maximum volume of oxygen uptake.

The determining the type of exercise may include calculating the basal metabolism of the user based on the user's gender, height, and weight input in advance in accordance with the user, calculating the activity metabolism of the user by applying the determined type of exercise and intensity of the exercise to the calculated basal metabolism, and determining the duration of the exercise based on the calculated activity metabolism.

In order to accomplish the above-described object, the present invention proposes a computer program recorded on a storage medium in order to execute a method of recommending exercise suitable for a user's real-time health condition based on an electrocardiogram. The computer program may be coupled to a computing device, including memory, a transceiver, an input/output device, and a processor for processing instructions loaded into the memory. Furthermore, the computer program may be a computer program recorded on a storage medium in order to execute obtaining, by the processor, an electrocardiogram signal measured for a user, identifying, by the processor, the state of the cardiac pump of the user by analyzing the electrocardiogram signal using pre-trained artificial intelligence, and determining, by the processor, the type of exercise to be recommended to the user in accordance with the identified state of the cardiac pump.

Specific details of other embodiments are included in the detailed description and the drawings.

**Advantageous Effects**

According to embodiments of the present invention, exercise optimized for a user's health condition at that time may be recommended simply by the user measuring an electrocardiogram.

The effects of the present invention are not limited to the effect mentioned above, and other effects not mentioned will be clearly understood by those skilled in the art, to which the present invention pertains, from the description of the claims.

**Description of Drawings**

FIG. 1 is an exemplary diagram illustrating an exercise recommendation means according to one embodiment of the present invention;

FIG. 2 is an exemplary diagram illustrating an exercise recommendation system according to one embodiment of the present invention;

FIG. 3 is a diagram of the logical configuration of an exercise recommendation server according to one embodiment of the present invention;

FIG. 4 is an exemplary diagram illustrating an artificial neural network (ANN) according to one embodiment of the present invention;

FIGS. 5 and 6 are exemplary diagrams illustrating processes of segmenting an electrocardiogram signal according to some embodiments of the present invention;

FIG. 7 is an exemplary diagram illustrating processes of determining a state value according to some embodiments of the present invention;

FIG. 8 is an exemplary diagram illustrating a process of identifying the state of a cardiac pump according to one embodiment of the present invention;

FIG. 9 is a diagram of the hardware configuration of an exercise recommendation server according to one embodiment of the present invention; and

FIG. 10 is a flowchart illustrating an exercise recommendation method according to one embodiment of the present invention.

**Mode for Invention**

It should be noted that the technical terms used herein are used only to describe specific embodiments and are not intended to limit the present invention. Furthermore, unless specifically defined otherwise herein, the technical terms used herein should be interpreted as having meanings generally understood by a person having ordinary skill in the art to which the present invention pertains, but should not be interpreted in an excessively comprehensive or excessively narrow sense. Furthermore, when the technical terms used herein are incorrect technical terms that do not accurately represent the spirit of the present invention, they should be replaced with and understood as technical terms that can be correctly understood by a person skilled in the art. Furthermore, the general terms used herein should be interpreted according to the definitions of dictionaries or according to the context, but should not be interpreted in an excessively narrow sense.

In addition, the singular expressions used herein include plural expressions unless the context clearly indicates otherwise. In the present application, the terms "include" or "have" should not be construed as necessarily including all of the various components or various steps described herein, and some of the components or steps may not be included or one or more additional components or steps may be included.

In addition, the terms including ordinal numbers, such as first, second, etc., used herein may be used to describe various components, but the components should not be limited by the terms. The terms are each used only for the purpose of distinguishing one component from another. For example, without departing from the scope of the present invention, the first component may be named the second component, and similarly, the second component may also be named the first component.

When a component is referred to as being "connected" or "coupled" to another component, it may be directly connected or coupled to the other component, but there may be another component therebetween. In contrast, when a component is referred to as being "directly connected" or "directly coupled" to another component, it should be understood that there are no other component therebetween.

Preferred embodiments according to the present invention will be described in detail with reference to the accompanying drawings below. Regardless of drawing numbers, identical or similar components will be given the same reference numerals and redundant descriptions thereof will be omitted. Furthermore, in the description of the present invention, when it is determined that a detailed description of a related known technology may obscure the gist of the present invention, the detailed description will be omitted. Furthermore, it should be noted that the accompanying drawings are only intended to facilitate the easy understanding of the spirit of the present invention and the spirit of the present invention should not be construed as being limited by the accompanying drawings. The spirit of the present invention should be construed as extending to all modifications, equivalents, and substitutes as well as the accompanying drawings.

As described above, conventional services that recommend or suggest exercise only recommend or suggest exercise in the order from exercise having a low impact on a user's body to exercise having the highest impact on the body, or simply recommend or suggest exercise that corresponds to age, gender, and chronic disease. However, the capacity of the heart, which is the factor that has the greatest influence on exercise ability, may change from moment to moment depending on a previously performed action, metabolism, stress, and/or the like.

In order to overcome these limitations, the present invention proposes a means for recommending exercise suitable for a user's real-time health condition.

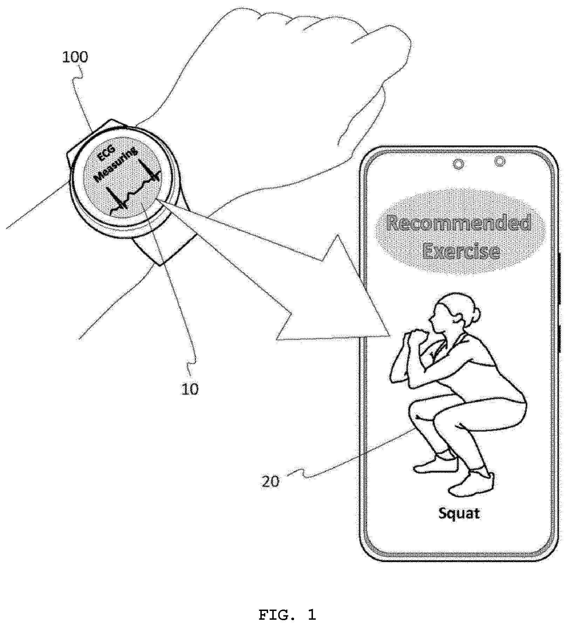

FIG. 1 is an exemplary diagram illustrating an exercise recommendation means according to one embodiment of the present invention.

As shown in FIG. 1, the exercise recommendation means according to one embodiment of the present invention may measure a user's electrocardiogram 10 using an electrocardiogram reader 100, may analyze the user's measured electrocardiogram 10 using artificial intelligence, may identify the state of a cardiac pump and/or the like according to the user's real-time health condition, and may immediately recommend exercise 20 corresponding to the identified state of a cardiac pump and/or the like to the user.

Accordingly, according to one embodiment of the present invention, the user may receive a recommendation for the exercise optimized for the user's health condition at that time simply by measuring the electrocardiogram 10.

Apparatuses and methods for implementing the above-described features will be specifically described below.

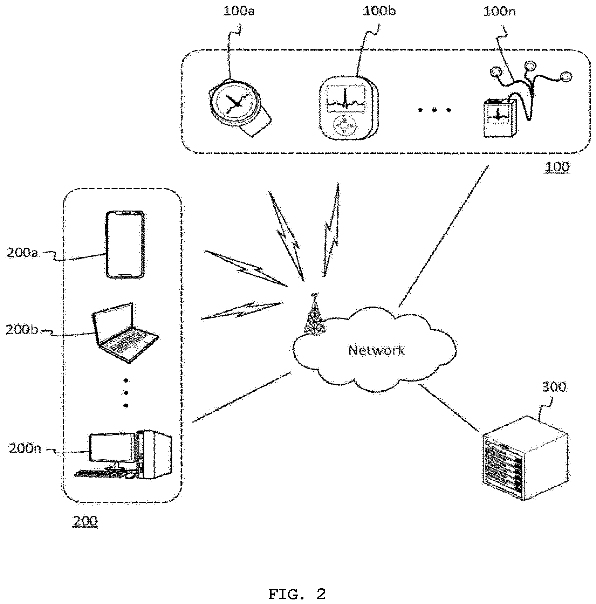

FIG. 2 is an exemplary diagram illustrating an exercise recommendation system according to one embodiment of the present invention.

As shown in FIG. 2, the exercise recommendation system according to the one embodiment of the present invention may be configured to include one or more electrocardiogram readers 100a, 100b, ···, and 100n (100), one or more pieces of user equipment 200a, 200b, ···, and 200n (200), and an exercise recommendation server 300.

The components of the exercise recommendation system according to the one embodiment of the present invention are merely functionally distinct components, so that two or more components may be implemented as an integrated component in an actual physical environment or one component may be implemented as separate components in an actual physical environment.

As for the individual components, each of the electrocardiogram readers 100 is a device capable of measuring and recording a user's electrocardiogram 10.

More specifically, each of the electrocardiogram readers 100 may induce changes in electric potential attributable to the electrical activity of the heart muscles via electrodes in contact with the user's body, may amplify the induced changes in electric potential, and may record them as waves.

The electrocardiogram reader 100 according to one embodiment of the present invention may induce an electrocardiogram according to any one of standard limb leads, unipolar limb leads, and precordial leads. The electrocardiogram (10) induction method of the electrocardiogram reader 100 is not limited thereto. The electrocardiogram reader 100 may be equipped with electrodes for a 1 lead, 6 leads, or 12 leads, and the number of leads of the electrocardiogram reader 100 is not limited thereto. Furthermore, the electrocardiogram reader 100 may have any one of the watch type 100a, the portable type 100b, and the holter type 100n, and the type of electrocardiogram reader 100 is not limited thereto.

An electrocardiogram signal (i.e., a recorded waveform signal) measured by the electrocardiogram reader 100 may be directly transmitted to the exercise recommendation server 300 through the network function of the electrocardiogram reader 100 itself, or may be input to the exercise recommendation server 300 via a third means. For example, the third means may be any one of a removable medium, the user equipment 200, and a data input action performed by a user, but is not limited thereto.

As the next component, the user equipment 200 may output information about the exercise determined by the exercise recommendation server 300.

More specifically, the user equipment 200 may receive information about exercise 20 from the exercise recommendation server 300. Furthermore, the user equipment 200 may output the received information about exercise 20. The information about exercise 20 received by the user equipment 200 from the exercise recommendation server 300 may include at least one of the type of exercise, the intensity of exercise, and the duration of exercise, but is not limited thereto.

The user equipment 200 according to one embodiment of the present invention is not limited to user equipment (UE) defined by the 3rd Generation Partnership Project (3GPP) or a mobile station (MS) defined by the Institute of Electrical and Electronics Engineers (IEEE). Any device may be accepted as the user equipment 200 as long as the device can transmit and receive data to and from the exercise recommendation server 300 and perform operations based on the transmitted and received data.

For example, the user equipment 100 may be, but is not limited to, any one of fixed computing devices such as a desktop 200c, a workstation, and a server, and mobile computing devices such as a smartphone 200a, a laptop 200b, a tablet, a phablet, a portable multimedia player (PMP), a personal digital assistant PDA, and an e-book reader.

As the next component, the exercise recommendation server 300 may identify the state of a cardiac pump and/or the like according to the user's real-time health condition based on the electrocardiogram 10 measured by the electrocardiogram reader 100, and may transmit information about exercise 20 corresponding to the identified state of the cardiac pump and/or the like to the user equipment 200.

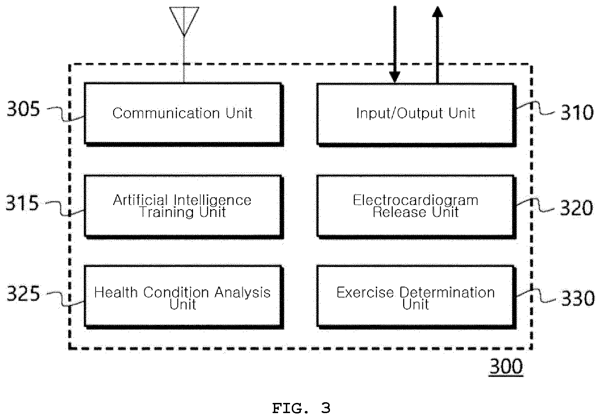

The specific components and operations of the exercise recommendation server 300 will be described later with reference to FIGS. 3 to 10.

The one or more electrocardiogram readers 100, the one or more pieces of user equipment 200, and the exercise recommendation server 300 that constitute the exercise recommendation system described above may transmit and receive data using a network, formed by combining one or more of a secure line, a public wired communication network, and a mobile communication network, that directly connects the devices.

For example, the public wired communication network may include, but is not limited to, Ethernet, Digital Subscriber Line (xDSL), Hybrid Fiber Coax (HFC), and Fiber To The Home (FTTH) networks. Furthermore, the mobile communication network may include, but is not limited to, Code Division Multiple Access (CDMA), Wideband CDMA (WCDMA), High Speed Packet Access (HSPA), Long Term Evolution (LTE), and 5th generation mobile telecommunication networks.

The components of the exercise recommendation server 300 having the features described above will be specifically described below.

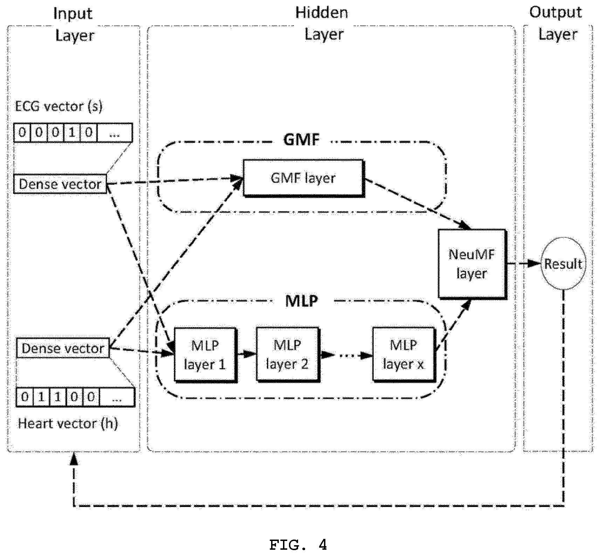

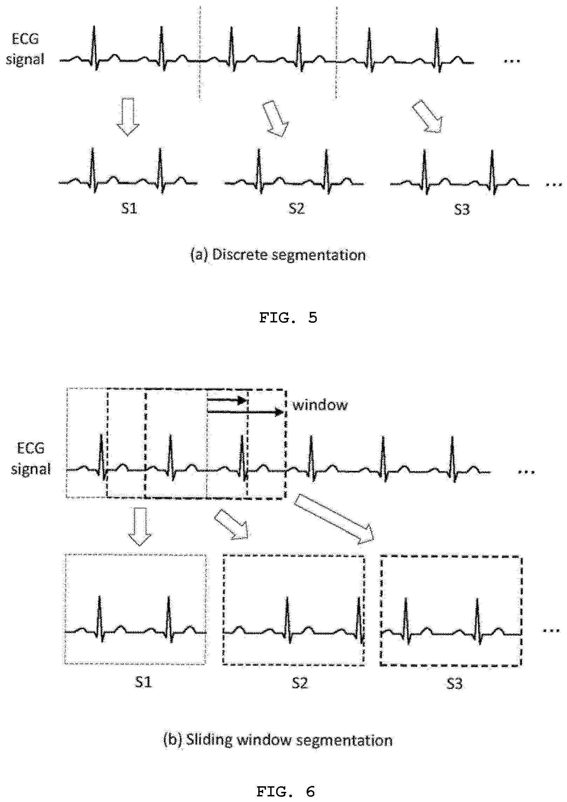

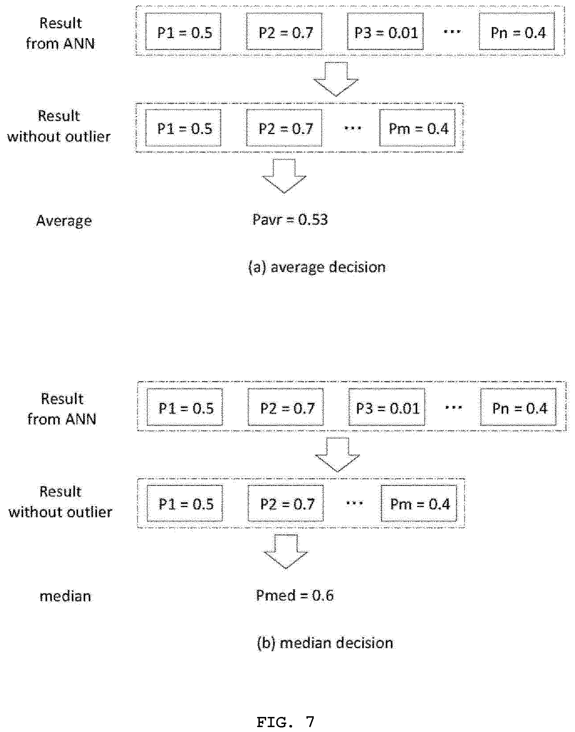

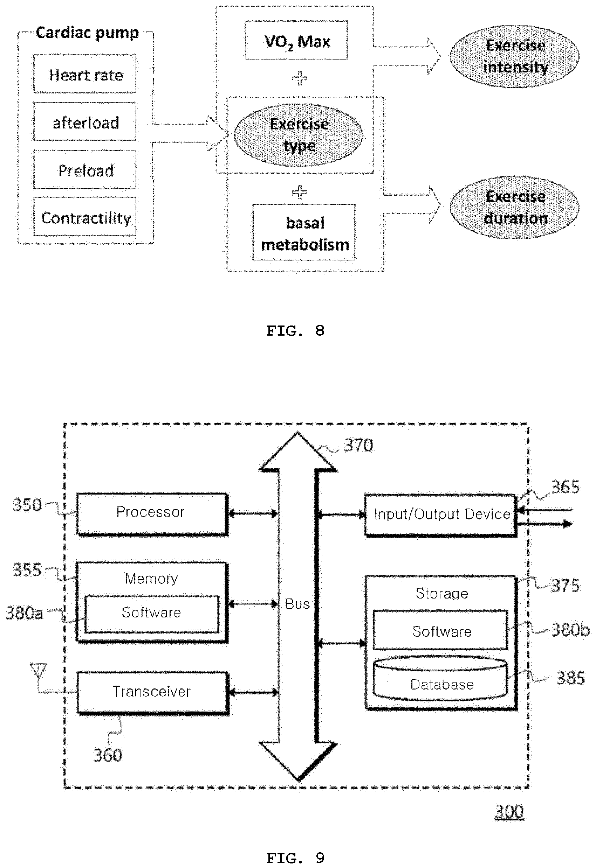

FIG. 3 is a diagram of the logical configuration of an exercise recommendation server according to one embodiment of the present invention. FIG. 4 is an exemplary diagram illustrating an artificial neural network (ANN) according to one embodiment of the present invention. FIGS. 5 and 6 are exemplary diagrams illustrating processes of segmenting an electrocardiogram signal according to some embodiments of the present invention. FIGS. 5 and 6 are exemplary diagrams illustrating processes of segmenting an electrocardiogram signal according to some embodiments of the present invention. FIG. 7 is an exemplary diagram illustrating processes of determining a state value according to some embodiments of the present invention. Furthermore, FIG. 8 is an exemplary diagram illustrating a process of identifying the state of a cardiac pump according to one embodiment of the present invention.

As shown in FIG. 3, the exercise recommendation server 300 according to one embodiment of the present invention may be configured to include a communication unit 305, an input/output unit 310, an artificial intelligence training unit 315, an electrocardiogram release unit 320, a health condition analysis unit 325, and an exercise determination unit 330.

The components of the exercise recommendation server 300 according to the one embodiment of the present invention are merely functionally distinct components, so that two or more components may be implemented as an integrated component in an actual physical environment or one component may be implemented as separate components in an actual physical environment.

As for the individual components, the communication unit 305 may transmit and receive data to and from the electrocardiogram reader 100 and the user equipment 200.

More specifically, the communication unit 305 may receive an electrocardiogram (10) signal from the electrocardiogram reader 100. Furthermore, the communication unit 305 may transmit information about exercise 20 to the user equipment 200. In this case, the information about exercise 20 may include, but is not limited to, one or more of the type of exercise, the intensity of exercise, and the duration of exercise.

As the next component, the input/output unit 310 may receive commands from an administrator or output operation results via a user interface (UI).

More specifically, the input/output unit 310 may receive the size of a window, which is a criterion for the release of an electrocardiogram (10) signal. The input/output unit 310 may receive a validated range, which is a criterion for the removal of outliers from probability values obtained from artificial intelligence. The input/output unit 310 may receive an exercise dictionary in which the states of a heart pump, the volumes of oxygen uptake, and activity metabolisms, and the types of exercise, the intensities of exercise, and the durations of exercise are matched with one another.

Meanwhile, the input/output unit 310 may receive an activation function to be applied to the GMF layer of an artificial neural network (ANN), an activation function to be applied to the MLP layer thereof, a weight, and a bias.

Furthermore, the input/output unit 310 may output a user's electrocardiogram (10) signal. The input/output unit 310 may also output information about exercise 20 to be recommended to the user.

As the next component, the artificial intelligence training unit 315 may train an artificial intelligence for identifying the state of a cardiac pump corresponding to a user's health condition based on an electrocardiogram (10) signal. Furthermore, the artificial intelligence training unit 315 may train artificial intelligence for identifying the maximum volume of oxygen uptake ($VO_2$ max) corresponding to a user's health condition based on an electrocardiogram (10) signal.

In this case, the state of the cardiac pump is a state value indicating the ability of the heart to circulate blood. This state of the cardiac pump may include heart rate, preload, afterload, and myocardial contractility. Furthermore, the maximum volume of oxygen uptake is the maximum amount of oxygen that the body can consume per unit time.

More specifically, the artificial intelligence, which is the training target of the artificial intelligence training unit 315, may be basically configured to include four artificial neural networks (ANNs).

A first ANN is an ANN for preload corresponding to the end diastolic volume of the heart. A second ANN is an ANN for afterload corresponding to the resistance that the heart receives while contracting. A third ANN is an ANN for the myocardial contractility of the heart. Furthermore, a fourth ANN is an ANN for the maximum volume of oxygen uptake.

According to one embodiment, the artificial intelligence training unit 315 may train the first ANN based on the displacements for the P wave, P-Q wave, and RQS wave included in each of a plurality of segmentation signals obtained by the segmentation of each electrocardiogram signal. The artificial intelligence training unit 315 may train the second ANN based on the displacements for the QRS wave, S-T wave, and T wave included in each of a plurality of segmentation signals obtained by the segmentation of each electrocardiogram signal. Furthermore, the artificial intelligence training unit 315 may train the third and fourth ANNs based on the displacements for all the waveforms included in each of a plurality of segmentation signals obtained by the segmentation of each electrocardiogram signal.

Each of the first ANN, the second ANN, the third ANN, and the fourth ANN, which are training targets of the artificial intelligence training unit 315, may be configured to include an input layer, a hidden layer, and an output layer.

In particular, as shown in FIG. 4, the hidden layers of the first ANN to the fourth ANN according to one embodiment of the present invention may each be configured in a form in which a generalized matrix factorization (GMF) and a multilayer perceptron (MLP) are mixed.

In other words, the hidden layer of the first ANN may include a GMF for the learning of the linearity relation present between the features of electrocardiogram signals (i.e., the features of the displacements for the P wave, the P-Q wave, and the RQS wave) and the states of preload, and an MLP for the learning of the nonlinearity relation present between the features of electrocardiogram signals and the states of preload. The hidden layer of the second ANN may include a GMF for the learning of the linearity relation present between the features of electrocardiogram signals (i.e., the features of the displacements for the QRS wave, the S-T wave, and the T wave) and the states of afterload, and an MLP for the learning of the nonlinearity relation present between the features of electrocardiogram signals and the states of afterload. The hidden layer of the third ANN may include a GMF for the learning of the linearity relation present between the features of electrocardiogram signals (i.e., the features of the displacements for all the waveforms) and the states of myocardial contractility, and an MLP for the learning of the nonlinearity relation present between the features of electrocardiogram signals and the states of myocardial contractility. Furthermore, the hidden layer of the fourth ANN may include a GMF for the learning of the linearity relation present between the features of electrocardiogram signals (i.e., the features of the displacements for all the waveforms) and the maximum volumes of oxygen uptake, and an MLP for the learning of the nonlinearity relation present between the features of electrocardiogram signals and the maximum volumes of oxygen uptake.

More specifically, the artificial intelligence training unit 315 may extract features of at least one electrocardiogram signal based on the displacements of the P wave, the P-Q wave, and the QRS wave according to the feature extraction criteria set in advance. The artificial intelligence training unit 315 may represent the extracted features of the electrocardiogram signal and the state of the preload as vectors by one-hot encoding them, and may generate dense vectors having reduced dimensions by embedding the vector for the features of the electrocardiogram signal and the vector for the state of the preload. The artificial intelligence training unit 315 may train the first ANN by inputting the Hadamard product of the dense vector for the features of the electrocardiogram signal and the dense vector for the state of the preload to the GMF layer of the hidden layer and also inputting the concatenation of the vector for the features of the electrocardiogram signal and the vector for the state of the preload to the MLP layer of the hidden layer.

The artificial intelligence training unit 315 may extract features of at least one electrocardiogram signal based on the displacements of the QRS wave, the S-T wave, and the T wave according to feature extraction criteria set in advance. The artificial intelligence training unit 315 may represent the extracted features of the electrocardiogram signal and the state of the preload as vectors by one-hot encoding them, and may generate dense vectors having reduced dimensions by embedding the vector for the features of the electrocardiogram signal and the vector for the state of the preload. The artificial intelligence training unit 315 may train the second ANN by inputting the Hadamard product of the dense vector for the features of the electrocardiogram signal and the dense vector for the state of the preload to the GMF layer of the hidden layer and also inputting the concatenation of the vector for the features of the electrocardiogram signal and the vector for the state of the preload to the MLP layer of the hidden layer.

Furthermore, the artificial intelligence training unit 315 may extract features of at least one electrocardiogram signal based on the displacements of all the waveforms of the electrocardiogram signal according to the feature extraction criteria set in advance. The artificial intelligence training unit 315 may represent the extracted features of the electrocardiogram signal and the state of the preload as vectors by one-hot encoding them, and may generate dense vectors having reduced dimensions by embedding the vector for the features of the electrocardiogram signal and the vector for the state of the preload. The artificial intelligence training unit 315 may train the third and fourth ANNs by inputting the Hadamard product of the dense vector for the features of the electrocardiogram signal and the dense vector for the state of the preload to the GMF layer of the hidden layer and also inputting the concatenation of the vector for the features of the electrocardiogram signal and the vector for the state of the preload to the MLP layer of the hidden layer.

To this end, the GMF layer in each ANN according to one embodiment of the present invention may be implemented using Equation 1 below:

$$a\big(h^T(p_u \odot q_i)\big) \tag{1}$$

where $p_u$ may be the dense vector of the features of the electrocardiogram signal, $q_i$ may be the dense vector of the state of the cardiac pump (the maximum volume of oxygen uptake in the case of the fourth ANN), $p_u \odot q_i$ may be the Hadamard product between the dense vector of the features of the electrocardiogram signal and the dense vector of the state of the cardiac pump (the maximum volume of oxygen uptake in the case of the fourth ANN), $a$ may be the activation function of the ANN, $h$ may be the edge weights of the hidden layer, and $h^T$ may be the vector product of the edge weights.

A dense vector is a vector whose dimension is reduced by embedding a vector represented by one-hot encoding. In other words, a dense vector is a vector whose dimension is reduced by removing values of 0 from a sparse vector represented by one-hot encoding.

Furthermore, the MLP layer in the ANN may be implemented using Equations 2 and 3 below:

$$\varphi_2(z_1) = a_2(W_2^T z_1 + b_2) \tag{2}$$

$$\sigma\big(h^T \varphi_L(z_{L-1})\big) \tag{3}$$

where $p_u$ is the dense vector of the features of the electrocardiogram signal, $q_i$ is the dense vector of the state of the cardiac pump (the maximum volume of oxygen uptake in the case of the fourth ANN), z is a concatenation of the dense vector of the features of the electrocardiogram signal and the dense vector of the state of the cardiac pump (the maximum volume of oxygen uptake in the case of the fourth ANN), $a$ is the activation function of the ANN, $W$ is the weight of each layer constituting the MLP, $W^T$ is the vector product of the weights, $b$ is the bias of each layer constituting the MLP, L is the number of layers constituting the hidden layer, and $\sigma$ is the sum of the progression.

Furthermore, each of the ANNs may be configured in the form of a recurrent neural network having a recursive connection structure in which the probability value output to the output layer is added to the vector for the state of the cardiac pump and then re-input to the input layer.

In this manner, the ANNs of the artificial intelligence training unit 315 each have a form in which GMF and MLP are mixed, so that not only the linearity relation present between the features of the electrocardiogram signal and the state of the cardiac pump (the maximum volume of oxygen uptake in the case of the fourth ANN) but also the nonlinearity relation can be machine-learned at the same time.

Referring back to FIG. 3 to describe the next component, the electrocardiogram release unit 320 may release the electrocardiogram (10) signal measured by the electrocardiogram reader 100.

More specifically, the electrocardiogram release unit 320 may obtain the electrocardiogram (10) signal measured for a user. That is, the electrocardiogram release unit 320 may directly receive the electrocardiogram (10) signal from the electrocardiogram reader 100 via the communication unit 305, or may directly receive the electrocardiogram (10) signal via the input/output unit 310.

The electrocardiogram release unit 320 may segment the electrocardiogram (10) signal into a plurality of segmentation signals according to the time-series order measured by the user.

Basically, as shown in FIG. 5, the electrocardiogram release unit 320 may segment the electrocardiogram (10) signal into a plurality of segmentation signals S1, S2, S3, ... by discretely cutting the electrocardiogram (10) signal at a predetermined size without overlap areas. In this case, the size for the discretely cutting of the electrocardiogram (10) signal may be a specific value set in advance by the input/output unit 310, but may not be limited thereto and may be a randomized value.

In contrast, as shown in FIG. 6, the electrocardiogram release unit 320 may segment the electrocardiogram (10) signal into a plurality of segmentation signals S1, S2, S3, ... having a size corresponding to that of the window by cutting the electrocardiogram (10) signal while sliding the window through the electrocardiogram (10) signal along the time axis. In this case, the size of the window for the cutting of the electrocardiogram (10) signal may be a specific value set in advance by the input/output unit 310.

Meanwhile, the electrocardiogram (10) signal may include the noise generated by a user's behavior during a measurement process. Accordingly, the electrocardiogram release unit 320 may remove the noise included in the plurality of segmentation signals S1, S2, S3, ....

More specifically, the electrocardiogram release unit 320 may identify the displacements of P waves, P-Q waves, QRS waves, S-T waves, and T waves included in each of the plurality of segmentation signals S1, S2, S3, .... The electrocardiogram release unit 320 may generate a normal distribution of the displacements of the identified P waves, P-Q waves, QRS waves, S-T waves, and T waves. Furthermore, the electrocardiogram release unit 320 may selectively remove only segmentation signals, having the displacements of P waves, P-Q waves, QRS waves, S-T waves, or T waves included in a preset noise range, from the generated normal distribution.

Referring back to FIG. 3 to describe the next component, the health condition analysis unit 325 may identify the state of the cardiac pump corresponding to the user's health condition by analyzing the electrocardiogram (10) signal by using artificial intelligence. Furthermore, the health condition analysis unit 325 may additionally identify the maximum volume of oxygen uptake corresponding to the user's health condition by analyzing the electrocardiogram (10) signal by using artificial intelligence.

First, the health condition analysis unit 325 may obtain a plurality of probability values P1, P2, P3, ..., Pn from the artificial intelligence by inputting the plurality of segmentation signals S1, S2, S3, **...,** obtained by segmentation via the electrocardiogram release unit 320, to the artificial intelligence.

The artificial intelligence according to one embodiment of the present invention is configured to include the first ANN to the fourth ANN, so that the health condition analysis unit 325 may input the plurality of segmentation signals S1, S2, S3, ... to each of the first ANN to the fourth ANN.

More specifically, the health condition analysis unit 325 may extract one or more features based on the displacements of the P wave, P-Q wave, and QRS wave included in each of the plurality of segmentation signals S1, S2, S3, ... according to the feature extraction criteria set in advance. The electrocardiogram release unit 320 may generate a vector of electrocardiogram signal features by one-hot encoding the extracted one or more features. The electrocardiogram release unit 320 may generate a dense vector having a reduced dimension by embedding the generated vector of electrocardiogram signal features. The electrocardiogram release unit 320 may input the generated dense vector to the GMF and MLP layers of the first ANN.

The health condition analysis unit 325 may extract one or more features based on the displacements of the QRS wave, S-T wave, and T wave included in each of the plurality of segmentation signals S1, S2, S3, ... according to the feature extraction criteria set in advance. The electrocardiogram release unit 320 may generate a vector of the features of the electrocardiogram signal by one-hot encoding the one or more extracted features. The electrocardiogram release unit 320 may generate a dense vector having a reduced dimension by embedding the generated vector of the features of the electrocardiogram signal. The electrocardiogram release unit 320 may input the generated dense vector to the GMF and MLP layers of the second ANN.

The health state analysis unit 325 may extract one or more features based on the displacements of all the waveforms included in each of the plurality of segmentation signals S1, S2, S3, **...** according to the feature extraction criteria set in advance. The electrocardiogram release unit 320 may generate a vector of the features of the electrocardiogram signal by one-hot encoding the one or more extracted features. The electrocardiogram release unit 320 may generate a dense vector having a reduced dimension by embedding the generated vector of the features of the electrocardiogram signal. The electrocardiogram release unit 320 may input the generated dense vector to the GMF and MLP layers of the third and fourth ANNs.

In addition, the health condition analysis unit 325 may obtain a plurality of probability values P1, P2, P3, ..., Pn corresponding to the plurality of segmentation signals S1, S2, S3, **...** from each of the first ANN to the fourth ANN.

Thereafter, the health condition analysis unit 325 may identify the state of the cardiac pump of the user based on a plurality of probability values P1, P2, P3, ..., Pn obtained from the first ANN to third ANN. Furthermore, the health condition analysis unit 325 may identify the maximum volume of oxygen uptake of the user based on a plurality of probability values P1, P2, P3, ..., Pn obtained from the fourth ANN.

More specifically, the health condition analysis unit 325 may identify the user's heart rate based on the number of waveforms included in the electrocardiogram signal per unit time. The health condition analysis unit 325 may determine the state value of the preload based on a plurality of probability values P1, P2, P3, ..., Pn obtained from the first ANN. The health condition analysis unit 325 may determine the state value of the afterload based on a plurality of probability values P1, P2, P3, ..., Pn obtained from the second ANN. The health condition analysis unit 325 may determine the state value of the myocardial contractility based on a plurality of probability values P1, P2, P3, ..., Pn obtained from the third ANN. Furthermore, the health condition analysis unit 325 may identify the state of the heart pump based on the identified user's heart rate, the state value of the preload, the state value of the afterload, and the state value of the myocardial contractility.

Furthermore, the health condition analysis unit 325 may identify the maximum volume of oxygen uptake of the user based on a plurality of probability values P1, P2, P3, ..., Pn obtained from the fourth ANN.

More specifically, as shown in FIG. 7, the health condition analysis unit 325 may remove outliers included in the plurality of probability values P1, P2, P3, ..., Pn obtained from each of the ANNs based on the validated range set in advance. The health condition analysis unit 325 may determine the average value Pavr of the plurality of probability values P1, P2, ..., Pm from which the outliers have been removed or the median value Pmed of the plurality of probability values P1, P2, ..., Pm from which the outliers have been removed to be a state value for the cardiac pump or the maximum volume of oxygen uptake.

That is, the health condition analysis unit 325 may remove outliers included in the plurality of probability values P1, P2, P3, ..., Pn obtained from the first ANN, and may determine the average value or median value of the plurality of probability values P1, P2, ..., Pm from which the outliers have been removed to be the state value of the preload. The health condition analysis unit 325 may remove outliers included in the plurality of probability values P1, P2, P3, ..., Pn obtained from the second ANN, and may determine the average value or median value of the plurality of probability values P1, P2, ..., Pm from which the outliers have been removed to be the state value of the afterload. The health condition analysis unit 325 may remove outliers included in the plurality of probability values P1, P2, P3, ..., Pn obtained from the third ANN, and may determine the average value or median value of the plurality of probability values P1, P2, ..., Pm from which the outliers have been removed to be the state value of the myocardial contractility. Furthermore, the health condition analysis unit 325 may remove outliers included in the plurality of probability values P1, P2, P3, ..., Pn obtained from the first ANN, and may determine the average value or median value of the plurality of probability values P1, P2, ..., Pm from which the outliers have been removed to be the maximum volume of oxygen uptake.

Referring back to FIG. 3 to describe the next component, the exercise determination unit 330 may determine the exercise to be recommended to the user based on the state of the cardiac pump and the maximum volume of oxygen uptake identified via the health condition analysis unit 325. The exercise determined by the exercise determination unit 330 may include one or more of the type of exercise, the intensity of the exercise, and the duration of the exercise.

As shown in FIG. 8, first, the exercise determination unit 330 may determine the type of exercise to be recommended to the user in accordance with the state of the cardiac pump identified via the health state analysis unit 325. To this end, the exercise determination unit 330 may determine the type of exercise (exercise type) matching the state of the cardiac pump of the user from the exercise dictionary set in advance. Furthermore, the exercise determination unit 330 may determine the intensity of exercise (exercise intensity) matching the maximum volume of oxygen uptake identified via the health condition analysis unit 325 and the previously determined type of exercise.

In addition, the exercise determination unit 330 may calculate the user's basal metabolism based on the user's gender, height, and weight input in advance in accordance with the user. The exercise determination unit 330 may calculate the user's activity metabolism by applying the determined type of exercise and intensity of exercise to the calculated basal metabolism. Furthermore, the exercise determination unit 330 may determine the duration of exercise (exercise duration) based on the calculated activity metabolism.

According to one embodiment of the present invention, the exercise determination unit 330 may identify the location of the user equipment 200 set in advance in accordance with the user. To this end, the exercise determination unit 330 may directly receive the location value of the user equipment 200 from the user equipment 200, or may receive the location value from the user. The exercise determination unit 330 may search for the altitude and weather condition corresponding to the identified location of the user equipment 200. Furthermore, the exercise determination unit 330 may apply a weight to the maximum volume of oxygen uptake, identified via the health condition analysis unit 325, in accordance with the found altitude and weather condition.

According to another embodiment of the present invention, the exercise determination unit 330 may determine the type of exercise by reflecting therein at least one of a time weight corresponding to the time at which the electrocardiogram (10) signal was measured and a human weight preset in accordance with the user.

In this case, the time weight may be a value set in accordance with the time and season at or in which the electrocardiogram (10) signal was measured. Furthermore, the human weight may be a value set in accordance with the state of the cardiac pump identified based on the electrocardiogram (10, ECG) signals measured for the user and a person registered as a friend.

In addition, the exercise determination unit 330 may transmit information about the determined exercise to the user equipment 200 set in advance in accordance with the user. In this case, the information about the exercise may include at least one of the type of exercise, the intensity of exercise, and the duration of exercise.

Hardware for implementing the logical components of the exercise recommendation server 300 having the features described above will be described in more detail below.

FIG. 9 is a diagram of the hardware configuration of an exercise recommendation server according to one embodiment of the present invention.

As shown in FIG. 9, the exercise recommendation server 300 according to the one embodiment of the present invention may be configured to include a processor 350, memory 355, a transceiver 360, an input/output device 365, a data bus 370, and storage 375.

More specifically, the processor 350 may implement the operations and functions of the exercise recommendation server 300 based on instructions according to software 380a implementing an exercise recommendation method that has been loaded into the memory 355.

The memory 355 may be loaded with software 380b implementing the exercise recommendation method that is stored in the storage 375.

The transceiver 360 may transmit and receive data to and from one or more of the electrocardiogram reader 100 and the user equipment 200.

The input/output device 365 may receive the signals required for the operation of the exercise recommendation server 300 or output operation results to the outside according to the commands of the processor 350.

The data bus 370 may be connected to the processor 350, the memory 355, the transceiver 360, the input/output device 365, and the storage 375, and may serve as a passage for the transmission of signals between the individual components.

The storage 375 may store application programming interfaces (APIs), library files, and resource files required for executing the software 380a implementing exercise recommendation methods according to various embodiments of the present invention. The storage 375 may store the software 380b implementing the exercise recommendation methods according to the various embodiments of the present invention. Furthermore, the storage 375 may include a database 385 for storing artificial intelligence training data, an exercise dictionary, and various setting values.

According to one embodiment of the present invention, the software 380a and 380b for implementing the exercise recommendation method loaded into the memory 355 or stored in the storage 375 may be a computer program stored on a storage medium in order to execute the step of obtaining, by the processor 350, an electrocardiogram (10) signal measured for a user via the transceiver 360 or input/output device 365, the step of identifying, by the processor 350, the state of the cardiac pump of the user by analyzing the electrocardiogram (10) signal using pre-trained artificial intelligence, and the step of determining, by the processor 350, a type of exercise to be recommended to the user in accordance with the identified state of the cardiac pump.

More specifically, the processor 350 may be configured to include one or more of a Central Processing Unit (CPU), an Application-Specific Integrated Circuit (ASIC), a chipset, and a logic circuit, but is not limited thereto.

The memory 355 may be configured to include one or more of Read-Only Memory (ROM), Random Access Memory (RAM), flash memory, and a memory card, but is not limited thereto.

The input/output device 365 may be configured to include one or more of input devices such as buttons, switches, a keyboard, a mouse, a joystick, and a touch screen, and one or more of output devices such as a liquid crystal display (LCD), a light emitting diode (LED) display, an organic light emitting diode (OLED) display, an active matrix OLED (AMOLED) display, a printer, and a plotter, but is not limited thereto.

When the embodiments included herein are implemented as software, the above-described method may be implemented as modules (processes, functions, and/or the like) that perform the above-described functions. The individual modules may be loaded into the memory 355 and executed by the processor 350. The memory 355 may be present inside or outside the processor 350, and may be connected to the processor 350 via various means known to the public.

The individual components shown in FIG. 9 may be implemented by various means (e.g., hardware, firmware, software, or a combination thereof). When implemented by hardware, an embodiment of the present invention may be implemented by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, microcontrollers, microprocessors, and/or the like.

Furthermore, when implemented by firmware or software, one embodiment of the present invention may be implemented in the form of modules, procedures, functions, and/or the like that perform the functions or operations described above, and may be recorded on a storage medium that can be read via various computer means. In this case, the storage medium may include program instructions, data files, data structures, and the like alone or in combination.

The program instructions recorded on the storage medium may be those specifically designed and configured for the present invention, or may be those known and available to those having ordinary knowledge in the computer software industry. For example, the storage medium includes magnetic media such as a hard disk, a floppy disk, and magnetic tape, optical media such as compact disk read-only memory (CD-ROM) and a digital video disk (DVD), magneto-optical media such as a floptical disk, and hardware devices specifically configured to store and execute program instructions such as ROM, RAM, and flash memory.

Examples of the program instructions may include not only machine language codes such as those that are generated by a compiler, but also high-level language codes that can be executed by a computer using an interpreter or the like. Such hardware devices may be configured to operate as one or more pieces of software to perform the operations of the present invention, and vice versa.

The operation of the exercise recommendation server 300 described above will be specifically described below.

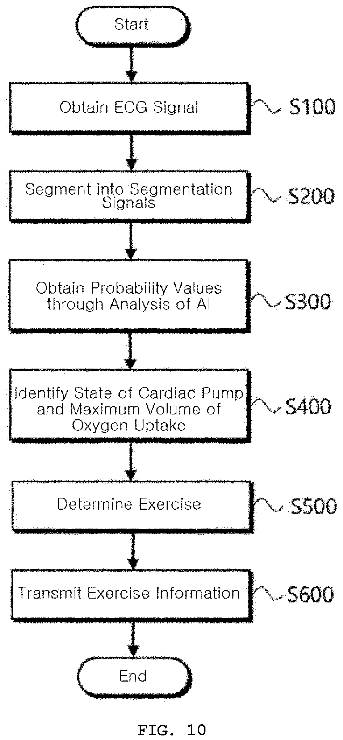

FIG. 10 is a flowchart illustrating an exercise recommendation method according to one embodiment of the present invention.

As shown in FIG. 10, the exercise recommendation server 300 according to one embodiment of the present invention may obtain an electrocardiogram (10) signal measured for a user in step S100.

More specifically, the exercise recommendation server 300 may directly receive the electrocardiogram (10) signal from the electrocardiogram reader 100, or may directly receive a signal from a user or another person.

Thereafter, the exercise recommendation server 300 may segment the electrocardiogram (10) signal into a plurality of segmentation signals S1, S2, S3, ... according to the time-series order measured by the user in step S200.

According to one embodiment, the exercise recommendation server 300 may segment the electrocardiogram (10) signal into a plurality of segmentation signals S1, S2, S3, ... by discretely cutting the electrocardiogram (10) signal at a predetermined size without overlap areas. According to another embodiment, the exercise recommendation server 300 may segment the electrocardiogram (10) signal into a plurality of segmentation signals S1, S2, S3, ... having a size corresponding to that of the window by cutting the electrocardiogram (10) signal while sliding the window through the electrocardiogram (10) signal along the time axis.

Meanwhile, the exercise recommendation server 300 may remove the noise included in the plurality of segmentation signals S1, S2, S3, ....

Thereafter, the exercise recommendation server 300 may obtain a plurality of probability values P1, P2, P3, ..., Pn by analyzing the plurality of segmentation signals S1, S2, S3, ... using the artificial intelligence in step S300.

More specifically, the exercise recommendation server 300 may extract one or more features based on the displacements of the P wave, P-Q wave, and QRS wave included in each of the plurality of segmentation signals S1, S2, S3, ... according to the feature extraction criteria set in advance. The exercise recommendation server 300 may generate a vector of the features of the electrocardiogram signal by one-hot encoding the extracted one or more features. The exercise recommendation server 300 may generate a dense vector having a reduced dimension by embedding the generated vector of the features of the electrocardiogram signal. The exercise recommendation server 300 may input the generated dense vector to the GMF and MLP layers of the first ANN.

The exercise recommendation server 300 may extract one or more features based on the displacements of the QRS wave, S-T wave, and T wave included in each of the plurality of segmentation signals S1, S2, S3, ... according to the feature extraction criteria set in advance. The exercise recommendation server 300 may generate a vector of the features of the electrocardiogram signal by one-hot encoding the one or more extracted features. The exercise recommendation server 300 may generate a dense vector having a reduced dimension by embedding the generated vector of the features of the electrocardiogram signal. The exercise recommendation server 300 may input the generated dense vector to the GMF and MLP layers of the second ANN.

The exercise recommendation server 300 may extract one or more features based on the displacements of all the waveforms included in each of the plurality of segmentation signals S1, S2, S3, ... according to the feature extraction criteria set in advance. The exercise recommendation server 300 may generate a vector of the features of the electrocardiogram signal by one-hot encoding the one or more extracted features. The exercise recommendation server 300 may generate a dense vector having a reduced dimension by embedding the generated vector of the features of the electrocardiogram signal. The exercise recommendation server 300 may input the generated dense vector to the GMF and MLP layers of the third and fourth ANNs.

In addition, the exercise recommendation server 300 may obtain a plurality of probability values P1, P2, P3, ..., Pn corresponding to the plurality of segmentation signals S1, S2, S3, ... from each of the first to fourth ANNs.

Thereafter, the exercise recommendation server 300 may identify the state of the cardiac pump of the user based on a plurality of probability values P1, P2, P3, ..., Pn obtained from the first to third ANNs, and may identify the maximum volume of oxygen uptake of the user based on a plurality of probability values P1, P2, P3, ..., Pn obtained from the fourth ANN in step S400.

More specifically, the exercise recommendation server 300 may remove outliers, included in the plurality of probability values P1, P2, P3, ..., Pn obtained from each of the ANNs, based on the preset validated range. The exercise recommendation server 300 may determine the average value Pavr of the plurality of probability values P1, P2, ..., Pm, from which the outliers have been removed or the median value Pmed of the plurality of probability values P1, P2, ..., Pm, from which the outliers have been removed, to be a state value for the cardiac pump or the maximum volume of oxygen uptake.

Thereafter, the exercise recommendation server 300 may determine the exercise to be recommended to the user based on the identified state of the cardiac pump and maximum volume of oxygen uptake in step S500. In this case, the determined exercise may include at least one of the type of exercise, the intensity of the exercise, and the duration of the exercise.

More specifically, the exercise recommendation server 300 may determine the type of exercise that matches the state of the cardiac pump of the user from the exercise dictionary set in advance. The exercise recommendation server 300 may determine the intensity of the exercise that matches the maximum volume of oxygen uptake and the previously determined type of exercise. Additionally, the exercise recommendation server 300 may calculate the user's activity metabolism by applying the previously determined type of exercise and intensity of the exercise to the user's basal metabolism calculated based on the user's gender, height, and weight input in advance in accordance with the user, and may determine the duration of the exercise based on the calculated activity metabolism.

Finally, the exercise recommendation server 300 may transmit information about the determined exercise to the user equipment 200 set in advance in accordance with the user in step S600.

As described above, although the preferred embodiments of the present invention have been disclosed in the present specification and the accompanying drawings, it is obvious to those skilled in the art that other modified examples based on the technical spirit of the present invention can be implemented in addition to the embodiments disclosed herein. Furthermore, although specific terms have been used in the present specification and the accompanying drawings, they have been used only in general senses to easily describe the technical content of the present invention and to help the understanding of the present invention, and are not intended to limit the scope of the present invention. Therefore, the detailed description above should not be construed as restrictive and should be considered as exemplary in all aspects. The scope of the present invention should be determined by a reasonable interpretation of the appended claims, and all changes within the equivalent scope of the present invention are included in the scope of the present invention.

**Claims**

1.   A method of recommending exercise, the method comprising:

obtaining an electrocardiogram signal measured for a user;
identifying a state of a cardiac pump of the user by analyzing the electrocardiogram signal using pre-trained artificial intelligence; and
determining a type of exercise to be recommended to the user in accordance with the identified state of the cardiac pump.

2.   The method of claim 1, wherein the identifying comprises:

segmenting the electrocardiogram signal into a plurality of segmentation signals according to a time-series order;
inputting the plurality of segmentation signals to the artificial intelligence, and obtaining a plurality of probability values from the artificial intelligence; and
identifying the state of the cardiac pump of the user based on the plurality of obtained probability values.

3.   The method of claim 2, wherein the artificial intelligence is configured to include a first artificial neural network (ANN) for preload corresponding to an end diastolic volume of a heart, a second ANN for afterload corresponding to resistance received by the heart while contracting, and a third ANN for myocardial contractility of the heart.

4.   The method of claim 3, wherein the identifying a state of a cardiac pump comprises:

identifying a heart rate based on a number of waveforms included in the electrocardiogram signal per unit time;
determining a state value of the preload based on a plurality of probability values obtained from the first ANN;
determining a state value of the afterload based on a plurality of probability values obtained from the second ANN;
determining a state value of the myocardial contractility based on a plurality of probability values obtained from the third ANN; and
identifying the state of the cardiac pump based on the heart rate, the state value of the preload, the state value of the afterload, and the state value of the myocardial contractility.

5.   The method of claim 3, wherein:

the first ANN is trained based on displacements for a P wave, P-Q wave, and QRS wave included in each of the plurality of segmentation signals;
the second ANN is trained based on displacements for a QRS wave, S-T wave, and T wave included in each of the plurality of segmentation signals; and
the third ANN is trained based on displacements for all waveforms included in each of the plurality of segmentation signals.

6. The method of claim 1, wherein the identifying a state of a cardiac pump comprises identifying the user's maximum volume of oxygen uptake by analyzing the electrocardiogram signal using the artificial intelligence.

7. The method of claim 6, wherein the determining a type of exercise comprises determining the type of exercise matching the state of the cardiac pump of the user from a previously set exercise dictionary, and determining an intensity of the exercise matching the determined type of exercise and the maximum volume of oxygen uptake.

8. The method of claim 7, wherein the determining a type of exercise comprises calculating a basal metabolism of the user based on the user's gender, height, and weight input in advance in accordance with the user, calculating an activity metabolism of the user by applying the determined type of exercise and intensity of the exercise to the calculated basal metabolism, and determining a duration of the exercise based on the calculated activity metabolism.

9. The method of claim 1, further comprising, after the determining a type of exercise, transmitting information, including the determined type of exercise, to user equipment (UE) set in advance in accordance with the user.

10. A computer program recorded on a storage medium, which is coupled to a computing device, including:

 memory;
 a transceiver;
 an input/output device; and
 a processor for processing instructions loaded into the memory, in order to execute:

  obtaining, by the processor, an electrocardiogram signal measured for a user;
  identifying, by the processor, a state of a cardiac pump of the user by analyzing the electrocardiogram signal using pre-trained artificial intelligence; and
  determining, by the processor, a type of exercise to be recommended to the user in accordance with the identified state of the cardiac pump.

FIG. 1

FIG. 2

305 — Communication Unit

310 — Input/Output Unit

315 — Artificial Intelligence Training Unit

320 — Electrocardiogram Release Unit

325 — Health Condition Analysis Unit

330 — Exercise Determination Unit

300

FIG. 3

FIG. 4

(a) Discrete segmentation

FIG. 5

(b) Sliding window segmentation

FIG. 6

Result
from ANN

| P1 = 0.5 | P2 = 0.7 | P3 = 0.01 | ⋯ | Pn = 0.4 |

Result
without outlier

| P1 = 0.5 | P2 = 0.7 | ⋯ | Pm = 0.4 |

Average

$Pavr = 0.53$

(a) average decision

Result
from ANN

| P1 = 0.5 | P2 = 0.7 | P3 = 0.01 | ⋯ | Pn = 0.4 |

Result
without outlier

| P1 = 0.5 | P2 = 0.7 | ⋯ | Pm = 0.4 |

median

$Pmed = 0.6$

(b) median decision

FIG. 7

FIG. 8

FIG. 9

```
        ┌─────────────┐
        │    Start    │
        └──────┬──────┘
               │
               ▼
     ┌──────────────────────┐
     │   Obtain ECG Signal  │  ∿ S100
     └──────────┬───────────┘
                │
                ▼
     ┌──────────────────────┐
     │ Segment into         │  ∿ S200
     │ Segmentation Signals │
     └──────────┬───────────┘
                │
                ▼
     ┌──────────────────────┐
     │ Obtain Probability   │  ∿ S300
     │ Values through       │
     │ Analysis of AI       │
     └──────────┬───────────┘
                │
                ▼
     ┌──────────────────────┐
     │ Identify State of    │  ∿ S400
     │ Cardiac Pump and     │
     │ Maximum Volume of    │
     │ Oxygen Uptake        │
     └──────────┬───────────┘
                │
                ▼
     ┌──────────────────────┐
     │ Determine Exercise   │  ∿ S500
     └──────────┬───────────┘
                │
                ▼
     ┌──────────────────────┐
     │ Transmit Exercise    │  ∿ S600
     │ Information          │
     └──────────┬───────────┘
                │
                ▼
        ┌─────────────┐
        │     End     │
        └─────────────┘
```

FIG. 10

| INTERNATIONAL SEARCH REPORT | International application No. |
|---|---|
| | **PCT/KR2023/010755** |

**A. CLASSIFICATION OF SUBJECT MATTER**

**G16H 20/30**(2018.01)i; **G16H 50/50**(2018.01)i; **G16H 50/20**(2018.01)i; **G16H 50/70**(2018.01)i; **A61B 5/349**(2021.01)i; **A61B 5/024**(2006.01)i; **A61B 5/22**(2006.01)i; **G06N 3/045**(2023.01)i; **G06N 3/08**(2006.01)i

According to International Patent Classification (IPC) or to both national classification and IPC

**B. FIELDS SEARCHED**

Minimum documentation searched (classification system followed by classification symbols)

G16H 20/30(2018.01); A61B 5/00(2006.01); G06F 3/0481(2013.01); G06N 3/02(2006.01); G06Q 50/22(2012.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심전도(ElectroCardioGram, ECG), 인공지능(artificial intelligence, AI), 운동 (exercise), 추천(recommendation), 심장 펌프(cardiac pump)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2013-0007463 A (KOREA ADVANCED INSTITUTE OF SCIENCE AND TECHNOLOGY) 18 January 2013 (2013-01-18)<br>See paragraphs [0014]-[0015], [0048], [0090] and [0098]-[0109], claims 4-5 and figures 3-4. | 1,2,6-10 |
| A | | 3-5 |
| Y | KR 10-2019-0019397 A (CHUNGBUK PROVINCIAL COLLEGE INDUSTRY ACADEMIC COOPERATION FOUNDATION) 27 February 2019 (2019-02-27)<br>See paragraphs [0057], [0063]-[0068] and [0111]-[0112] and figures 3-7. | 1,2,6-10 |
| Y | KR 10-2330705 B1 (HYUN, Dae Jin) 25 November 2021 (2021-11-25)<br>See paragraph [0053], claim 7 and figure 2. | 8 |
| Y | KR 10-2022-0032678 A (SWALLABY) 15 March 2022 (2022-03-15)<br>See paragraphs [0055] and [0068] and claims 1-2 and 5. | 1,9,10 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 November 2023** | **07 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/010755**

**C.     DOCUMENTS CONSIDERED TO BE RELEVANT**

| Category* | Citation of document, with indication, where appropriate, of the relevant passages | Relevant to claim No. |
|---|---|---|
| Y | KR 10-2022-0091779 A (LG ELECTRONICS INC.) 01 July 2022 (2022-07-01)<br>See paragraphs [0051] and [0097] and claims 1 and 7. | 1,9,10 |
| Y | KR 10-2016-0061007 A (MEDI PLUS SOLUTION CO., LTD.) 31 May 2016 (2016-05-31)<br>See claims 1-2 and 6-7. | 1,9,10 |

Form PCT/ISA/210 (second sheet) (July 2022)

INTERNATIONAL SEARCH REPORT
Information on patent family members

International application No.

**PCT/KR2023/010755**

| Patent document cited in search report | | | Publication date (day/month/year) | Patent family member(s) | | | Publication date (day/month/year) |
|---|---|---|---|---|---|---|---|
| KR | 10-2013-0007463 | A | 18 January 2013 | KR | 10-1398542 | B1 | 27 May 2014 |
| | | | | WO | 2013-002568 | A2 | 03 January 2013 |
| | | | | WO | 2013-002568 | A3 | 11 April 2013 |
| KR | 10-2019-0019397 | A | 27 February 2019 | KR | 10-2033065 | B1 | 16 October 2019 |
| KR | 10-2330705 | B1 | 25 November 2021 | WO | 2021-242023 | A1 | 02 December 2021 |
| KR | 10-2022-0032678 | A | 15 March 2022 | KR | 10-2548357 | B1 | 28 June 2023 |
| KR | 10-2022-0091779 | A | 01 July 2022 | None | | | |
| KR | 10-2016-0061007 | A | 31 May 2016 | None | | | |

Form PCT/ISA/210 (patent family annex) (July 2022)